# EUROPEAN PATENT APPLICATION

(11) **EP 4 104 786 A1**
(43) Date of publication of application: **21.12.2022**
(21) Application number: 21179450.8
(22) Date of filing: 15.06.2021
(51) Int. Cl.: A61B 34/20, A61B 90/00, A61B 34/10

(54) **TECHNIQUE FOR DETERMINING POSES OF TRACKED VERTEBRAE**

(71) Applicant: Stryker European Operations Limited, Carrigtwohill, Co. Cork T45 HX08 (IE)
(72) Inventor: GHANAM, Fadi, 79227 Schallstadt (DE)
(74) Representative: Röthinger, Rainer

(57) **Abstract**

A tracker system for determining poses of at least two vertebrae and a computer-implemented method of using the tracker system are presented. The tracker system comprises a first tracker trackable in 5 degrees of freedom, DOF, and attachable to a first vertebra and a second tracker trackable in 5 DOF and attachable to a second vertebra. In some variants, the first and second tracker each comprises an elongated body and two imageable markers that are attached to the elongated body and spaced apart from each other along a length of the elongated body. A tracking coordinate system is registered in 6 DOF with an image coordinate system associated with first image data taken by a medical imaging system and indicative of the first and second vertebra. The method comprises receiving intraoperative tracking data and determining, from the received intraoperative tracking data, tracker poses of the first tracker and the second tracker in 5 DOF. Further still, the method comprises determining, from the tracker poses and based on the registration of the tracking coordinate system with the image coordinate system, poses of the first vertebra and the second vertebra in 5 DOF.

## Description

### Technical Field

The present disclosure generally relates to the field of surgical tracking. In particular, a computer-implemented method of determining poses of tracked vertebrae is presented. Also presented are a computer program product and a data processing system configured to perform the method, and a tracking system for determining the poses.

### Background

Different surgical tracking techniques are used for assisting a surgeon or controlling operation of a surgical robot. For example, medical image data of a patient may be visualized on a display and overlaid with a model, position or trajectory of a handheld surgical tool tracked by a tracking system. As another example, an arm of a robot holding a surgical tool may be navigated relative to a tracked bony structure such as a vertebra.

Especially in the field of spinal surgery, it is mandatory that tracking and navigation operations are performed at a high degree of accuracy, since any surgical error may result in damaging the spinal cord. In some spinal interventions, the placement of pedicle screws for example is facilitated by a tracker attached to the patient. Image data of the tracker are acquired via a camera in the operating room and registered with image data of the spine acquired via a (e.g., pre-operative) medical imaging procedure such as computer tomography (CT). The accuracy in navigated screw placement typically decreases the further away the surgical site gets from the tracker. This decrease in accuracy results from movement and changes in the anatomy during the surgery after the initial registration, e.g., due to the patient breathing or due to the screw placement itself. To reduce patient movement, the breathing frequency of the patient is often reduced during spinal surgery. However, this approach may put the patient at health risks.

Different approaches are known for compensating movements and changes in the anatomy during spinal surgery. For example, the registration may be executed repeatedly. Also, the tracker initially attached to a particular vertebra may be relocated and re-registered to another vertebra the surgeon is currently working on. Alternatively, as disclosed in EP 3 369 394 A, a bone pin with a surveillance marker may be utilized for monitoring a change of a distance between the tracker and the surveillance marker, indicating a movement and thus a need for re-registration. Repeated registrations are time consuming and lead to prolonged surgeries.

US 2019/002975 A discloses providing pedicle screws with two imageable markers, which allows for easy computation of a screw trajectory during screw placement as well as tracking of a vertebra after the placement of at least two pedicle screws or of a pedicle screw and a pin with one imageable marker in the vertebra.

US 10,485,617 B discloses attaching a tracking array with four imageable markers to a spinous process of a vertebra. Due to their respective size, multiple such tracking arrays would obstruct vertebral access if attached to each of multiple adjacent vertebrae.

### Summary

There is a need for a technique for efficiently determining poses of two tracked vertebrae.

According to a first aspect, a computer-implemented method of determining poses of at least two vertebrae of a patient when a first tracker trackable in 5 degrees of freedom, DOF, is attached to a first vertebra and a second tracker trackable in 5 DOF is attached to a second vertebra is provided. A tracking coordinate system is registered in 6 DOF with an image coordinate system associated with first image data taken by a medical imaging system and indicative of the first and second vertebra. The method comprises receiving intraoperative tracking data, determining, from the intraoperative tracking data, tracker poses of the first tracker and the second tracker in 5 DOF, and determining, from the tracker poses and based on the registration of the tracking coordinate system with the image coordinate system, poses of the first vertebra and the second vertebra in 5 DOF.

The first image data may have been taken preoperatively or intraoperatively. The medical imaging system may be any suitable imaging device, e.g., an X-ray scanner, an magnetic resonance imaging (MRI) scanner or a CT scanner. The second image data may be taken intraoperatively.

In some variants, the 5 DOF tracking provides an acceptable tradeoff between tracking accuracy (excluding the 6^{th} DOF) of the respective vertebrae and tracker size or surgical obstruction (only one tracker with two imageable markers per vertebra). Each of the first and second tracker may comprise an elongated body and the 5 DOF of the tracker poses may exclude a DOF pertaining to a respective rotation of the first tracker and the second tracker with regard to a rotational axis defined by the elongated body of the respective tracker.

According to a first realization, at least one of the first and second tracker is an electromagnetic tracker. In such a realization, the intraoperative tracking data may comprise data from a device capable of processing an output signal of the electromagnetic tracker(s). Each of the one or more electromagnetic trackers may in particular comprise a single coil trackable in 5 DOFs. Each electromagnetic tracker may be connected to a respective electromagnetic sensor. A position of a source of an electromagnetic field to be sensed by the electromagnetic tracker(s)may be known in the tracking coordinate system.

According to a second realization (that may be combined with the first realization), at least one of the first and second tracker comprises two imageable markers that are attached to the elongated body and spaced apart from each other along a length of the elongated body. The imageable markers may in particular be tracked optically. The intraoperative tracking data may comprise second image data taken by a camera of a tracking system and indicative of the imaged markers of the first tracker and the second tracker.

As understood herein, an imageable marker is a marker that is detectable in image data. The imageable marker may be detectable in image data that have been acquired using an optical, a magnetic or an X-ray based imaging technique.

According to one realization, the respective two markers have the same mutual arrangement for each of the first tracker and the second tracker, such that the first tracker and the second tracker cannot be differentiated in the image data solely by the imaged markers. The imaged markers may all have the same shape, e.g., a spherical, cubic or pyramidal shape. The markers may be active or passive markers. Active markers may configured to generate and emit electromagnetic radiation, and passive markers may be configured to reflect electromagnetic radiation.

According to another realization, a distance between the respective two markers along the length of the elongated body is different for the first tracker and the second tracker. Therefore, a respective tracker may be identified (e.g., by the tracking system) based on the distance between the respective two markers along the length of the elongated body of the respective tracker.

According to still another realization, at least one of the first tracker and the second tracker may comprise a divot. The divot may be configured to receive a tip of a screw (e.g., a pedicle screw). The method may further comprise determining a length of the screw based on a distance between one of the imaged markers of the first or second tracker receiving the tip of the screw and at least one imaged marker attached to an instrument holding the screw.

Registering the tracking coordinate system with the image coordinate system may comprise a 6 DOF registration that is based on using (at least) three imageable markers with a fixed spatial relation for the registration. The tracking coordinate system may belong to a tracking system that also comprises the first tracker and the second tracker. When registering the tracking coordinate system with the image coordinate system, or in a separate registration step, a 5 DOF registration between a dedicated tracker coordinate system associated with each of the trackers and a dedicated image data segment coordinate system associated with an image data segment of each tracked vertebra in the first image data may be performed.

According to one realization, the method may comprise defining a virtual 6 DOF tracker comprising at least three of the imageable markers of the first and second tracker that are imaged in third image data taken by the camera of the tracking system. The tracking coordinate system may be registered with the image coordinate system using the at least three imageable markers of the virtual 6 DOF tracker as imaged in the third image data. The registration of the tracking coordinate system with the image coordinate system may comprise one or multiple transformations (one or more translations and/or one or more rotations) and may be a part of the series of multiple known transformations for determining the poses of the vertebra. The third image data may be taken intraoperatively.

According to another realization, a 6 DOF reference tracker is provided that has a fixed relation to the patient (e.g., has been attached to particular vertebra). The 6 DOF tracker may comprise at least three imageable markers that are imaged in fourth image data taken by the camera of the tracking system. The tracking coordinate system may be registered with the image coordinate system using the at least three imageable markers of the 6 DOF reference tracker as imaged in the fourth image data. The fourth image data may be taken intraoperatively. The 6 DOF reference tracker may be a tracker comprising four imageable markers.

The method may further comprise determining a change of the pose of at least one of the first and the second tracker relative to a pose of the reference tracker. Such a change, once determined, may be employed in different ways (e.g., visualized to the surgeon, trigger a new registration procedure, etc.).

According to one realization, the pose of at least one of the first tracker and the second tracker in 5 DOF is determined in the tracking coordinate system. The determination of the pose of at least one of the first tracker and the second tracker in 5 DOF in the tracking coordinate system may be based on a known position of the camera that has taken the second image data.

The method may further comprise determining a change of the pose of the first tracker relative to a pose of the second tracker. Such a change, once determined, may be employed in different ways (e.g., visualized to the surgeon, trigger a new registration procedure, etc.).

The first and second tracker may each comprise an optically, in particular visually detectable identification characteristic. The identification characteristics of the first and second tracker may be distinguishable from each other. The method may further comprise identifying (e.g., by the tracking system) at least one of the first tracker and the second tracker based on its identification characteristic.

The identification characteristics may comprise an optically detectable surface characteristic of the first tracker and second tracker, in particular of the respective elongated body. The optically detectable surface characteristics may comprise different colors and/or different patterns (e.g., red, blue, green, yellow, and/or a stripes or dots). Also, multiple distinguishable features may be used simultaneously for further facilitating tracker identification. The identification characteristics may be used to facilitate a fast and easy identification of the respective tracker either automatically or by a surgeon. Thus, the cognitive load on the surgeon may be reduced. In some variants, errors due to an erroneous identification of a particular tracker may be prevented.

At least one of the first tracker and the second tracker may comprise an attachment member. The attachment member can be configured to attach the respective tracker to a vertebra or a vertebral implant, such as a pedicle screw. The attachment member may be configured for being mounted at or in a spinous process of one of the vertebrae. Additionally, or in the alternative, the attachment member may comprise a clamp or a screw having an extension that is collinear with an extension of the elongated body. Tracker attachment may be done in preparation for a subsequent navigation procedure, e.g., placing a pedicle screw.

The method may further comprise defining, based on the determined poses of the first vertebra and the second vertebra in 5 DOF, a trajectory configured for guiding a surgical tool (e.g., for guiding a screw driving tool along the trajectory during pedicle screw placement). The trajectory may be visualized to a surgeon relative to the first and second vertebrae.

The second image data that is received during the method may be received and processed in real-time. Thus, the method may enable dynamic tracking of the vertebrae having one of the first and second tracker attached thereto.

The method may comprise visualizing the determined pose of the first and second vertebra. The visualizing may comprise obtaining a first image data segment including the first vertebra and a second image data segment including the second vertebra, and arranging the first image data segment relative to the second image data segment based on the determined poses of the first vertebra and the second vertebra in 5 DOF. The image segments may be overlaid over intra-operatively acquired medical image data (acquired, e.g., using a cone beam CT technique, MRI, or ultrasound). The visualizing may be performed in real-time on a display device.

A computer program product is also provided. The computer program product comprises instructions configured to perform the steps of the method presented herein when the computer program product is executed on one or more processors.

Also provided is a data processing system. The data processing program comprises a processor configured to perform the steps of the method for any realization of the method presented herein.

According to a further aspect, a tracker system for determining the poses of at least two vertebrae of a patient is provided. The system comprises a first tracker attachable to a first vertebra and a second tracker attachable to a second vertebra. The first and second tracker each comprises an elongated body and two imageable markers that are attached to the elongated body and spaced apart from each other along a length of the elongated body. The respective two markers have the same mutual arrangement for each of the first tracker and the second tracker, such that the first tracker and the second tracker cannot be differentiated in image data solely by the imaged markers. The first and second tracker each comprises an optically detectable identification characteristic. The optically detectable identification characteristics of the first and second tracker are optically distinguishable from each other.

According to one realization, the tracker system may comprise a reference tracker attachable to the patient. The reference tracker may comprise at least three imageable markers.

### Brief Description of the Drawings

Further features and advantages of the computer-implemented method and the system presented herein are described below with reference to the accompanying drawings, in which:
- Fig. 1A: illustrates a schematic representation of a tracker system for determining poses of multiple vertebrae of a patient;
- Fig. 1B: illustrates a schematic representation of optical trackers usable in the system of Fig 1A;
- Fig. 1C: illustrates a schematic representation of another optical tracker usable in the system of Fig 1A;
- Fig. 1D: illustrates a schematic representation of electromagnetic trackers attached to vertebrae;
- Fig. 2: illustrates a schematic representation of another tracker system for determining poses of multiple vertebrae of a patient;
- Fig. 3: illustrates a schematic representation of an exemplary tracking system for visualizing poses of multiple vertebrae of a patient;
- Fig. 4: illustrates a flow diagram of a method of determining poses of two vertebrae of the patient;
- Figs. 5A-5C: illustrate schematic representations of image data processing used in the context of the method of Fig. 4;
- Fig. 6: illustrates a schematic representation of a series of coordinate transformations for determining the tracker poses and vertebra poses according to a first realization of the method of Fig. 4;
- Fig. 7: illustrates a schematic representation of a series of coordinate transformations for determining the tracker poses and vertebra poses according to a second realization of the method shown in Fig. 4;
- Fig. 8A: illustrates a schematic representation of the visualization of the tracked poses of two vertebrae on a display device; and
- Fig. 8B: illustrates a schematic representation of the visualization of the two vertebrae shown in FIG.8A with individually adapted poses.

### Detailed Description

In the following description, for purposes of explanation and not limitation, specific details are set forth in order to provide a thorough understanding of the present disclosure. It will be apparent to one skilled in the art that the present disclosure may be practiced in other embodiments that depart from these specific details.

The same reference numerals are used to denote the same or similar components.

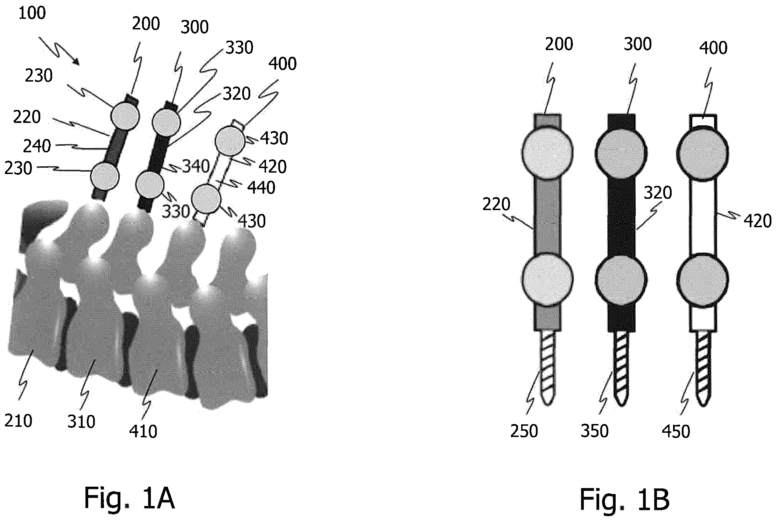

Fig. 1A illustrates a schematic representation of a tracker system 100 for determining poses of multiple vertebrae of a patient. The tracker system 100 comprises a first tracker 200 attached to a first vertebra 210. The first tracker 200 comprises an elongated body 220 and two imageable markers 230 that are attached to the elongated body 220 and spaced apart from each other along a length of the elongated body 220. The tracker system 100 further comprises a second tracker 300 attached to a second vertebra 310. Like the first tracker 200, the second tracker 300 comprises an elongated body 320 and two imageable markers 330 that are attached to the elongated body 320 and spaced apart from each other along a length of the elongated body 320. Each of the first tracker 200 and the second tracker 300 is configured to be tracked in 5 DOF.

The elongated bodies 220, 320 are rod- or bar-shaped and have, for example, a circular or elliptic cross-section. The markers 230, 330 are spherically shaped and symmetrically sit on the elongated bodies 220, 230. In other words, the elongated bodies 220, 230 extend through opposite poles of the spherically shaped markers 230, 330. The markers 230, 330 have the same diameters and the same surface characteristics. In some variants, the markers 230, 330 are configured to reflect electromagnetic radiation (e.g., in the infrared or visible spectrum) utilized by a tracking system that comprises the tracker system 100. In other words, the markers 230, 330 may be passive devices.

The respective two markers 230, 330 have the same mutual arrangement for each of the first tracker 200 and the second tracker 300. For example, they have the same distance from each other along the respective elongated member 220, 320. As such, the first tracker 200 and the second tracker 300 cannot be differentiated in image data solely by the imaged markers 230, 330. Such marker arrangement ensures that each tracker 200, 300 has the same (e.g., optical) properties. Further, each tracker 200, 300 can easily be replaced by another, identical tracker.

To still be able to distinguish the first and second tracker 200, 300 from each other, each of the first and second tracker 200, 300 comprises an optically detectable identification characteristic 240, 340. Those identification characteristics 240, 340 are optically distinguishable from each other. The optically detectable identification characteristics 240, 340 shown in Fig. 1A are different colors (e.g., red and blue) of the elongated bodies 220, 320 of the first and second tracker 200, 300. In other variants (not shown), the optically detectable identification characteristics 240, 340 may additionally or alternatively comprise optically distinguishable patterns, e.g., different stripes or dots on the elongated bodies 220, 320. As such, the first tracker 200 and the second tracker 300 can be differentiated in image data by determining their respective optically detectable identification characteristic 240, 340.

In other realizations, the respective two markers 230, 330 of the trackers 200, 300 have different mutual arrangements for each of the first tracker 200 and the second tracker 300 as different optically detectable identification characteristics 240, 340. For example, they have different distances from each other along the respective elongated member 220, 320. As such, the first tracker 200 and the second tracker 300 can be differentiated in image data by determining the distances between the imaged markers 230, 330.

The tracker system 100 shown in Fig. 1A further comprises an optional third tracker 400 analogously built to the first and second tracker 200, 300 (i.e., with an elongated body 420 and two markers 430 attached thereto). The third tracker 400 is attached to a third vertebra 410. The third tracker 400 is provided with an optically detectable identification characteristic 440 different from the optically detectable identification characteristics 240, 340 for the first and second tracker 200, 300.

The number of trackers 200, 300, 400 comprised by the system 100 may depend on the surgical needs and the preferences of a surgeon. Typically, one tracker 200, 300, 400 is attached to one vertebra 210, 310, 410, so that the number of trackers 200, 300, 400 may also depend on the number of vertebrae 210, 310, 410 to be treated during a spinal intervention. In some variants, two or more of the trackers 200, 300 400 may be attached to a single vertebra.

Fig. 1B illustrates a schematic representation of the three trackers 200, 300, 400 in the system of Fig 1A. Each of the trackers 200, 300, 400 comprises an attachment member 250, 350, 450 at one of its longitudinal ends. The attachment members 250, 350, 450 are configured as bone screws. Each of the screws 250, 350, 450 has an longitudinal extension that is collinear with a longitudinal extension of the respective elongated body 220, 320, 420. Each of the bone screws 250, 350, 450 is configured for being mounted in a spinous process of the associated vertebra 210, 310, 410 (see Fig. 1A). In other variants (not shown), the attachment members 250, 350, 450 may be bone clamps instead of bone screws. Using a clamp 250, 350, 450 instead of a screw 250, 350, 450 may reduce the time needed for attaching a tracker 200, 300, 400 to a vertebra 210, 310, 410.

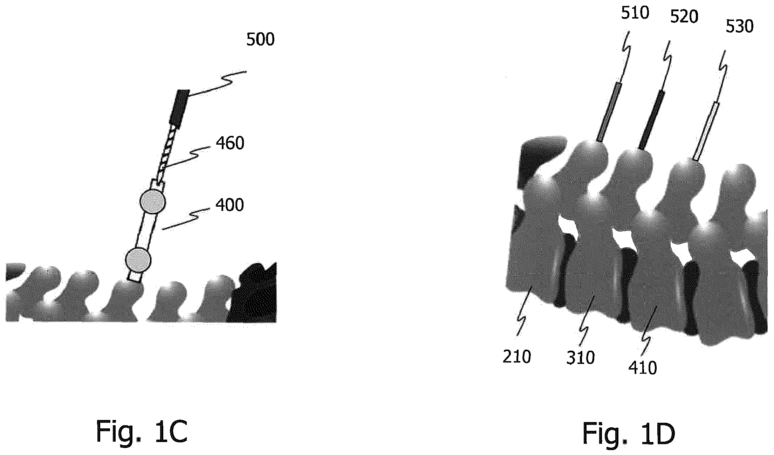

Fig. 1C illustrates a schematic representation of another tracker realization. The shown tracker 400 comprises, in addition or as an alternative to the features described above, a divot 460 at a longitudinal end of the elongated body 420 opposite to the end of the elongated body 420 with the attachment member 450. The divot 460 is configured to receive a tip of a screw, e.g., a pedicle screw, that is being held by a tool 500. The tool 500 may comprise one or more optically detectable markers (e.g., arranged on a detachable tracker, not shown) so that a distance between one of the markers of the tracker 400 with the divot 460 and the one or more markers of the tool 500 can be determined by a tracking system. In this way, a fast and easy way for verifying the length of the screw held by the tool 500 is provided.

Fig. 1D illustrates a schematic representation of three electromagnetic trackers 510, 520, 530 attached to vertebrae 210, 310, 410. Each of the shown trackers 510, 520, 530 comprises an elongated body with a single coil (not shown) capable of sensing an electromagnetic field. When one of the electromagnetic trackers 510, 520, 530 is moved within the electromagnetic field, an electric current is induced. The amplitude of the induced current depends on the relative pose of the respective coil to a source of the electromagnetic field (i.e., an electromagnetic field generator, not shown in Fig. 1D).

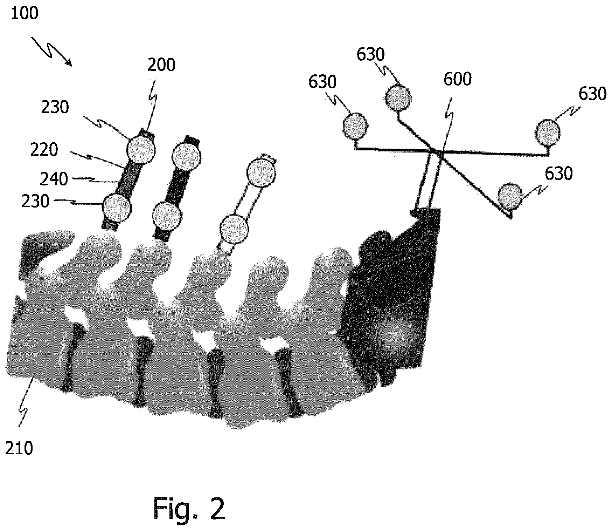

Fig. 2 illustrates a schematic representation of another realization of a tracker system 100 for determining poses of multiple vertebrae of a patient. The tracker system 100 of Fig. 2 differs from the system shown in Fig. 1A by additionally comprising a reference tracker 600 attached to a patient's pelvis or any of the patient's vertebrae. The reference tracker 600 comprises four imageable markers 630 that can be tracked in 6 DOF. In other embodiments (not shown), the reference tracker comprises three or more than four imageable markers 630. The imageable markers 630 of the reference tracker 600 may have the same or a different configuration (e.g., shape) than the imageable markers 230, 330, 430 of the trackers 200, 300 400 comprising two imageable markers 230, 330, 430.

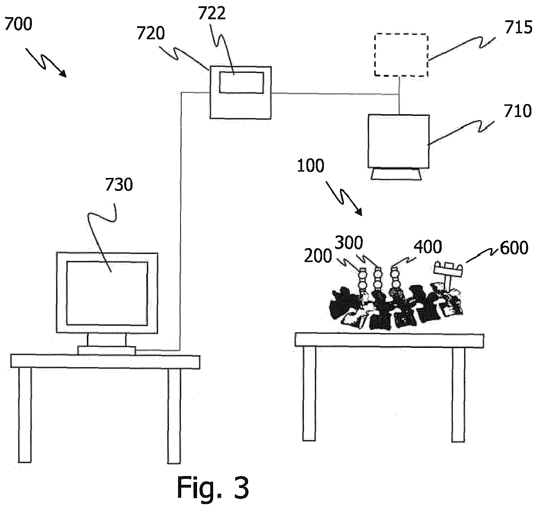

Fig. 3 illustrates a schematic representation of a tracking system 700 for tracking poses of at least two vertebrae of a patient in 5 DOF. The tracking system 700 comprises the tracker system 100 as discussed with reference to Fig. 2, but could alternatively comprise the tracker system 100 of Fig. 1A without the reference tracker 600, or any other tracker system.

The tracking system 700 further comprises a camera 710 for imaging the imageable markers 230, 330, 430, 630 of the trackers 200, 300, 400, 600. The camera 710 is configured to generate image data indicative of the imageable markers 230, 330, 430, 630. The image data may be generated as a stream of image data frames (e.g., at a frame rate between 200 Hz and 2 kHz). The imaged markers 230, 330, 430, 630 are detectable in the image data (e.g., by an image processing algorithm). In some variants, the camera 710 is a stereo camera.

While not illustrated in Fig. 3, the tracking system 700 may comprise a source of electromagnetic radiation (e.g., in the infrared spectrum) that floods the surgical site with the electromagnetic radiation. The emitted electromagnetic radiation is reflected by the imageable markers 230, 330, 430, 630 for detection by the camera 710. In case all of the trackers 200, 300, 400, 600 are configured as active devices (e.g., with the imageable trackers being configured as light emitting diodes), the source of electromagnetic radiation may be omitted.

Alternatively or in addition to one or more of the trackers 200, 300, 400, 600 comprising imageable markers 230, 330, 430, 630, one or more electromagnetic trackers 510, 520, 530 as shown in FIG. 1D can be used (not shown). In this case, a field generator (not shown) locally generates a field of electromagnetic radiation at the surgical site. A movement of each of the electromagnetic trackers 510, 520, 530 induces a current in its associated coil depending on the relative pose of the coil to the source of the electromagnetic radiation. The source of electromagnetic radiation may have a known position relative to the camera 710 of tracking system 700. The induced current is measured by a device 715 to which the tracker coils are connected via dedicated cables. The device 715, sometimes also called "EM Box", is capable of measuring the induced currents and processing the measurement results as needed to intraoperatively generate output data.

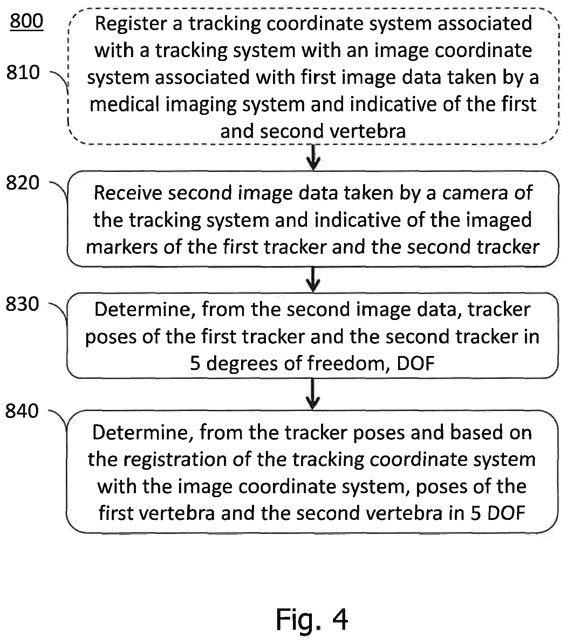

The tracking system 700 further comprises a data processing system 720 configured to receive and process at least one of image data from the camera 710 and output data from the device 715. The data processing system 720 comprises a processor 722 configured to perform the steps of any method realization of the present disclosure (e.g., as shown in Fig. 4 and discussed in detail with regard to Figs. 4 to 7 below). The data processing system 720 stores a computer program product (not shown) comprising instructions configured to control operation of the processor 722. In some variants, the data processing system 720 is built from cloud computing resources. In other variants, the data processing system 720 is physically located in the operating room.

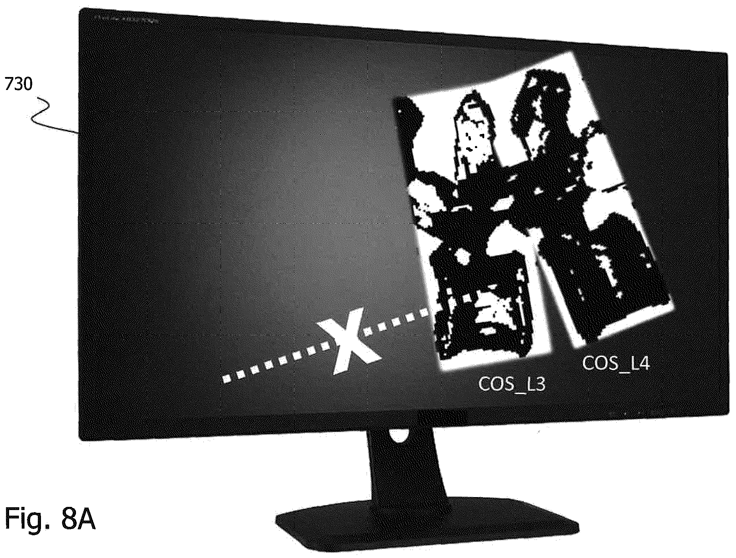

The tracking system 700 further comprises a display device 730 configured to receive processed image data from the data processing system 720 and visualize the received image data (e.g., as shown in Fig. 8A). The display device 730 is located in the field of view of a surgeon (not shown). The visualization takes place in real-time. In some variants, the visualization helps the surgeon to navigate a surgical tool relative to one of the patient's vertebrae. In such variants, the tracking system 700 provides navigation assistance.

In some variants of the present disclosure, the data processing system 720 is configured to generate control data for a surgical robot (not shown) based on the image data received from the camera 710. The control data are configured to control movement of a surgical tool attached to an arm of the surgical robot. In such variants, the surgical robot may navigate the surgical tool autonomously or selectively constrain movements of the surgical tool by a surgeon.

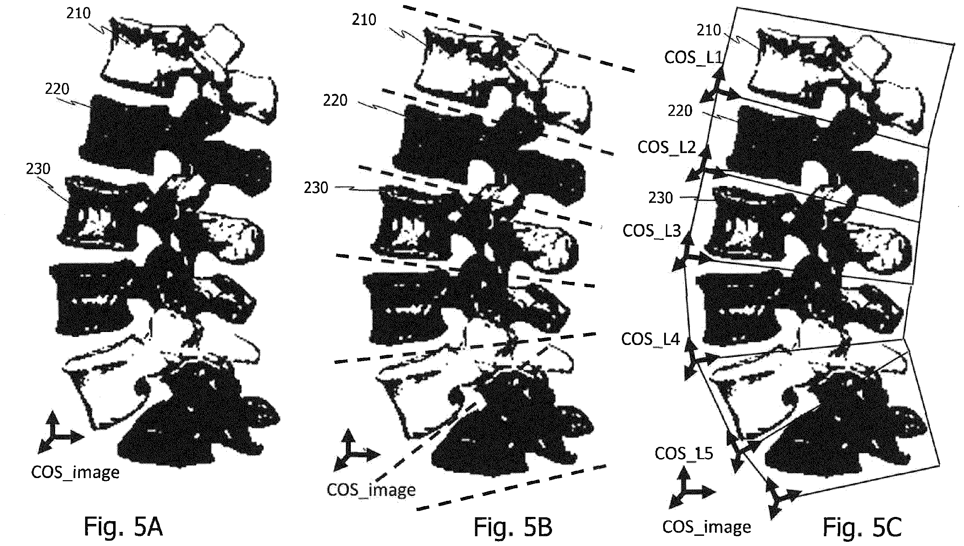

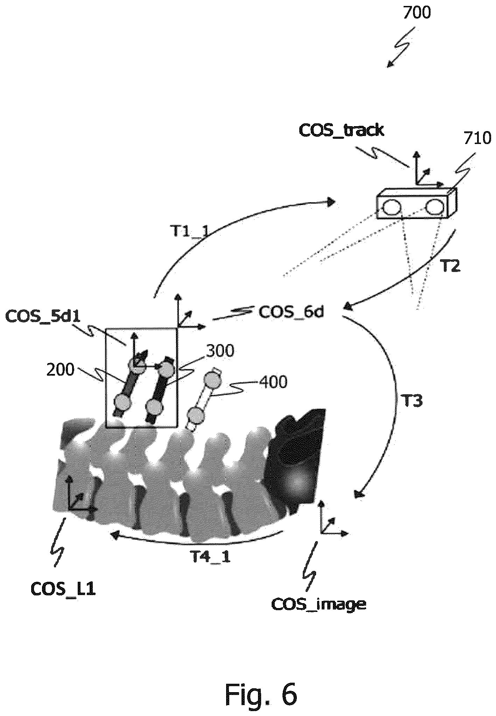

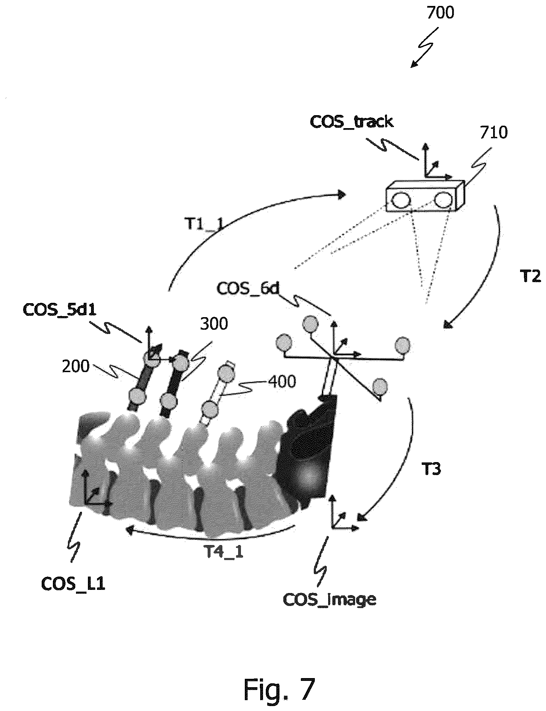

Fig. 4 illustrates a flow diagram 800 of a method of determining poses of two vertebrae 210, 310 of a patient when a first tracker 200 is attached to a first vertebra 210 and a second tracker 300 is attached to a second vertebra 310. The method may be performed by the data processing system 720 and in particular by the processor 722 and will be explained with reference to Figs. 5A to 5C and 6 to 8. Figs. 5A to 5C illustrate an exemplary image processing technique beneficial for providing surgical navigation assistance. Fig. 6 illustrates coordinate transformations according to a first implementation of the present disclosure that does not rely on a reference tracker 600 (see Fig. 1A), while Fig. 7 illustrates coordinate transformations according to a second implementation of the present disclosure that uses a reference tracker 600 (see Figs. 2 and 3). Fig. 8A illustrates an exemplary surgical navigation procedure.

In an optional, preparatory step 810 illustrated in Fig. 4, a tracking coordinate system (COS_track) associated with the tracking system 700 (and, e.g., located at a center of the camera 710) is registered with an image coordinate system (COS_image) associated with (first) image data taken by a medical imaging system (not shown) and indicative of the first and second vertebra 210, 310. This registration is done in 6 DOF. The image data may have been acquired pre-operatively or intra-operatively using a medical imaging technique of comparatively high resolution, such as CT or MRI. As an example, Fig. 5A illustrates a schematic visualization of three-dimensional CT image data of some of a patient's vertebrae.

The 6 DOF registration of COS_track with COS_image in step 810 involves a coordinate system (COS_6d, see Figs. 5 and 6) associated with at least three imageable markers having a spatially rigid relationship to each other (e.g., at least three out of the multiple imageable markers 230, 330, 430, 630 of the trackers 200, 300, 400, 600 in Figs. 1A and 2). The term COS_6d refers to the fact that, when combined, the at least three imageable markers are (at least temporarily and for registration purposes) trackable by the tracking system 700 in 6 DOF. The coordinate system COS_6d can be associated with image data taken by the camera 710 and being indicative of the at least three imageable markers having a spatially rigid relationship to each other.

When involving coordinate system COS_6d, the registration step 810 comprises two substeps (not shown). The first substep comprises determining a transformation T2 (as shown in Figs. 6 and 7) for transforming coordinates from COS_track to COS_6d. The second substep comprises determining a transformation T3 (as shown in Figs. 6 and 7) for transforming coordinates from COS_6d to COS_image. Determining transformation T3 may comprise identifying common features in the image data taken by the camera 710 and associated with COS_6d on the one hand and, on the other hand, in the image data taken by the medical imaging device (see Fig. 5A). The common features may be anatomical landmarks of the vertebrae 210, 310 or artificial markers (also called fiducials), like the tracker markers 230, 330. The identification of the common features may be performed manually by a surgeon (using, e.g., a tracked pointer device) or automatically, e.g., by using image recognition software. Based on the identified common features, the transformation T3 for transforming coordinates from COS_6d to COS_image (and/or an inverse transformation for the transformation coordinates from COS_image to COS_6d is determined). Applying the respective transformation on either COS_6d or COS_image results in receiving coordinates of both the first and second tracker 200, 300 and the first and second vertebra 210, 310 in a common coordinate system. Depending on the direction of the determined transformation, the common coordinate system is either COS_6d or COS_image.

In an optional step (not shown in Fig. 4), it may be determined which of the trackers 200, 300, 400 is attached to which vertebra 210, 310, 410 based on the registration step 810 (i.e., based on the tracker 200, 300, 400 and the vertebra 210, 310, 410 having coordinates associated with a common coordinate system, such as COS_6d or COS_image).

For this purpose, each of the respective tracker 200, 300, 400 may be identified manually and/or automatically in the image data of the camera 710 as used during registration based on the identification characteristics 240, 340, 440 (e.g., the different colors and/or patterns) of the elongated body 220, 320, 420 of the respective tracker 200, 300, 400. After identification, the coordinates for each identified tracker 200, 300, 400 are determined in the common coordinate system (e.g.,COS_6d or COS_image) based on the registration step 810.

Further, the image data associated with COS_image may be processed as shown in Figs. 5A to 5C. Fig. 5A illustrates pre-operatively acquired CT image data of the patient's vertebrae (including the vertebrae 210, 310, 410). In other variants, the image data of the vertebrae may be acquired intra-operatively, for example using cone beam CT (CBCT).

The image data representative of the vertebrae is automatically segmented per vertebra by initially separating the vertebrae from each other using, e.g., two-dimensional geometric structures indicated as dashed lines in Fig. 5B. In a next step shown in Fig. 5C, segmentation of the pre-operatively acquired CT image data includes the definition of a bounding volume per vertebra that is defined by the two-dimensional geometric structures towards its adjacent vertebrae as illustrated in Fig. 5B and a lateral enclosure dimensioned to include the complete image data of a given vertebra (the bounding volumes are only schematically illustrated in Fig. 5C by continuous lines). In an optional further segmentation step, a vertebra surface is determined within each bounding volume. The vertebra surface delimits a three-dimensional image data segment of the pre-operatively acquired CT image data for a given vertebra. As shown in Fig. 5C, each of those image data segments is associated with a local coordinate system defined as COS_Li (with i = 1, 2, 3...) based on a common nomenclature referring to vertebrae levels. Since the image data segments are segmented from image data associated with COS_image, a transformation between each COS_Li and COS_image can be determined such that the coordinates of the COS_Li can be determined in the common coordinate system (e.g., COS_6d or COS_image) of the registration step 810. Analogously, a transformation from COS_image to COS_Li can be determinded, which is denoted as transformation T4_i (with i = 1, 2, 3,...) referring to the COS_Li (shown in Figs. 6 and 7).

Finally, since the coordinates of the identified trackers 200, 300, 400 and the coordinate systems COS_Li are transformed into the common coordinate system (e.g., COS _6d or COS_image), each tracker can be related to a vertebra coordinate system COS_Li and thus to a vertebra , as illustrated in Fig. 5C.

Alternatively, it may be known which of the tracker 200, 300, 400 is attached to which vertebra 210, 310, 410 of the spine of the patient from attaching the tracker 200, 300, 400 to the vertebrae 210, 310, 410 or from image data indicative of each of the tracker 200, 300, 400 and the vertebrae 210, 310, 410 the tracker are attached to.

Returning to Fig.4, in step 820, (second) image data taken by the camera 710 of the tracking system 700 and indicative of the imaged markers 230, 330 of the first tracker 200 and the second tracker 200 are received by the data processing system 720 in real-time and on a temporarily continuous manner. As explained above, the image data may be received as a stream of image data frames.

In step 830, poses of the first tracker 200 and the second tracker 300 are determined by the data processing system 720 from the image data stream received in step 820. The data processing system 720 processes the image data stream in real-time to determine real-time poses of the first tracker 200 and the second tracker 300. Since each of the first and second tracker 200, 300 comprises two imageable markers 230, 330, the poses of the first tracker 200 and the second tracker 300 can be determined by the data processing system 720 in 5 DOF. The first and second tracker 200, 300 may be identified manually and/or automatically in the received image data, for example, based on the identification characteristics 240, 340.

The poses of the first tracker 200 and the second tracker 300 may be determined in the tracker coordinate system COS_track. For this purpose, a tracker coordinate system may be associated with each of the 5 DOF trackers 200, 300. In Figs. 6 and 7, this local tracker coordinate system is denoted COS_5di (with i = 1, 2 referring to the respective first and second tracker 200, 300). Further, a respective coordinate transformation denoted T1_i (with i = 1, 2 referring to the respective COS_5di) and shown in Figs. 6 and 7 may be applied to each tracker coordinate system COS_5di for transforming the coordinates of the respective COS_5di to the COS_track.

In step 840, poses of the first vertebra 210 and the second vertebra 310 are determined from the tracker poses determined in step 830 and based on the registration of the tracking coordinate system with the image coordinate system, i.e., based on the transformations determined in step 810. Since the tracker poses are determined in 5 DOF and the poses of the vertebra are determined from the tracker poses, the vertebra poses are determined in the same 5 DOF as the tracker poses.

In more detail, step 840 may comprise applying a sequence of the transformations T2 to T4_i determined during the registration step 810 and the image segmenting described with reference to Fig. 5 on the respective tracker poses determined in step 830, i.e., applying transformations T2 to T4_i on the respective COS_5di after the respective transformation T1_i was applied. Applying the transformations T2 and T3 provides the respective poses of the first and second tracker 200, 300 in the image coordinate system COS_image. Further applying the respective transformations T4_i then provides individual poses of the coordinate systems COS_Li associated with the first and second vertebra 210, 310.

Fig. 6 illustrates a schematic representation of a series of coordinate transformations for determining the poses of the first tracker 200 and the second tracker 300 as well as the poses of the first vertebra 210 and the second vertebra 310 from the tracker poses according to a first realization of the method 800 shown in Fig. 4. For illustration purposes, the tracker system 100 of Fig. 1A is shown together with the camera 710 of the tracking system 700 (see Fig. 3).

In Fig. 6, the series of transformations is used for transforming coordinates from one of the coordinate systems COS_5di associated with the respective tracker 200, 300, 400 trackable in 5 DOF to the coordinate system COS_Li associated with the vertebra 210, 310, 410 the respective tracker 200, 300, 400 is attached to. In the first transformation T1_i (with i = 1, 2, 3), the coordinates of the respective imaged markers 230, 330, 430 are transformed from the respective tracker coordinate system COS_5di into the coordinate system COS_track of the tracking system 700 (that, as said, may be centered at the camera 710). In the second transformation T2, the coordinates are transformed from COS_track into the coordinate system COS_6d associated with an (imaginary) reference tracker built from at least three of the imageable markers 230, 330 of the first and second tracker 200, 300 of Fig. 1A. The at least three imageable markers 230, 330 of the first and second tracker 200, 300 define a virtual 6 DOF tracker when being in a spatially rigid relationship to each other. In a third transformation T3, the coordinates are transformed from COS_6d to COS_image. In a fourth transformation T4, the coordinates are transformed from COS_image to the respective COS_Li.

In summary, coordinates from a local tracker coordinate system such as COS_5di (e.g., i = 1, 2) associated with, for example, one of the tracked first and second tracker 200, 300 are transformed to a coordinate system COS_Li (e.g., i = 1, 2) associated with the vertebra 210, 310 the respective tracker 200, 300 is attached to by applying the transformation T = T1_i ^{∗} T2 ^{∗} T3 ^{∗} T4_i. In other words, by applying the coordinate transformation T, a pose or change of a pose of one of the tracker coordinate systems COS_5di associated with the respective tracker 200, 300, 400 trackable in 5 DOF is transformed to a pose or change of a pose of the corresponding COS_Li associated with the vertebra 210, 310, 410 the respective tracker 200, 300, 400 is attached to.

Fig. 7 schematically illustrates a series of coordinate transformations for determining the poses of the first vertebra 210 and the second vertebra 310 from the tracker poses according to a second realization of the method shown in Fig. 4. In comparison to the realization shown in Fig. 6, the realization of Fig. 7 relies on the reference tracker 600 with at least three imageable markers 630. The coordinate system COS_6d is associated with the reference tracker 600. The transformation T is defined analogously to the workflow shown in Fig. 6, i.e., as T = T1_i^{∗}T2^{∗}T3^{∗}T4_i.

According to the realizations described above, determining the poses of the vertebra from the tracker poses and based on the registration of the tracking coordinate system with the image coordinate system comprise a series of one or more known coordinate transformations (e.g., a combination of one or more translations and/or one or more rotations).

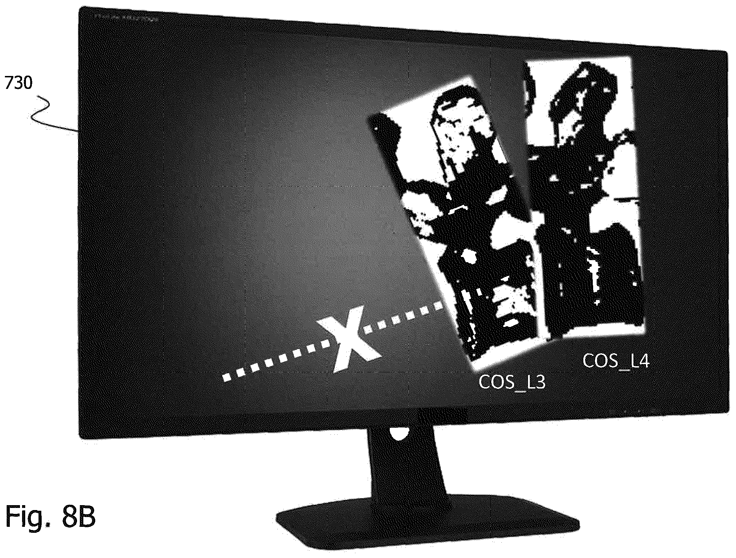

Figs. 8A and 8B illustrate a schematic representation of a visualization of the pose of two vertebrae 210, 310, 410 on the display device 730 of Fig. 3. This visualization can be performed based on the result of step 840 to provide navigation assistance to a surgeons. Additionally, or in the alternative, the result of step 840 may be used to generate a data set for control of a surgical robot.

With reference to the visualization of Fig. 8A, two of the image data segments, each associated with one respective vertebra coordinate system COS_Li as described with reference to Fig. 5, are overlaid over intra-operatively acquired medical image data (e.g., CBCT image data). For visualization purposes, the image data segments are oriented according to the 5 DOF vertebra poses as determined in step 840, i.e., the poses of the respective coordinate systems COS_Li (see Figs. 5 to 7). As shown in Fig. 8A, the labelling information may be visualized also, at least in the context of one or more of the image data segments that are associated with a tracked vertebra (here: COS_L3 and COS_L4).

The visualization of the 5 DOF vertebra poses in some variants comprises a plastic three-dimensional representation of the image segments and/or image data (e.g., the vertebrae 210, 310, 410 or parts thereof). Additionally, or as an alternative, the visualization comprises a two-dimensional (e.g., cross-sectional) representation thereof.

The respective 5 DOF vertebra poses may be updated continuously and in real time according to one of the realizations described with reference to Figs. 6 and 7. Continuously updating the vertebra poses comprises continuously updating the transformations T1_i and T4_i. The transformations T1_i and T4_i are updated each time a pose is updated. The visualization may then be updated continuously and in real time according to the updated vertebra poses. Updating the vertebra poses shown in Fig. 8A can comprise tracking one or both of the tracker 200, 300, 400 attached to the vertebrae 210, 310, 410 associated with COS_L3 and COS_L4 using the tracking system 700 (see Figs. 3, 6 and 7) and collectively or individually adapting their poses relative to each other in 5 DOF dependent on the tracking. FIG. 8B illustrates a schematic representation of the visualization of the two vertebrae shown in FIG. 8A with individually adapted 5DOF poses.The provision of the individual coordinate systems COS_5di for the respective tracker 200, 300, 400 and COS_Li for the respective vertebrae 210, 310, 410 the tracker 200, 300, 400 are attached to (see Figs. 5A to 5C, 6 and 7) facilitates the tracking of individual ones of the vertebrae 210, 310, 410 in 5 DOF and their corresponding visualization in 5 DOF. As such, the surgeon obtains valuable information about the intraoperative vertebra poses. Such information may in certain realizations additionally, or in the alternative, be processed by a surgical robot.

Further still, the navigation information may be augmented by tracking a surgical tool 500 (see Fig. 1C) and visualizing on the display device 730 a navigational aid indicative of the tracked surgical tool 500, such as a screw driver or a drill, relative to the visualized vertebrae 210, 310, 410. In the scenario illustrated in Fig. 8A, a trajectory and a position of a tool tip are tracked and visualized by a dashed line and a cross, respectively. The surgeon thus obtains visual information on the spatial relationship between the surgical tool 500 and a high-quality representation of the vertebrae 210, 310, 410 associated with the coordinate systems COS_L3 and COS_L4.

## Claims

1. A computer-implemented method (800) of determining poses of at least two vertebrae of a patient when
a first tracker (200) trackable in 5 degrees of freedom, DOF, is attached to a first vertebra (210) and a second tracker (300) trackable in 5 DOF is attached to a second vertebra (310),
wherein a tracking coordinate system is registered (810) in 6 DOF with an image coordinate system associated with first image data taken by a medical imaging system and indicative of the first and second vertebra (210, 310),
the method (800) comprising:
receiving (820) intraoperative tracking data;
determining (830), from the intraoperative tracking data, tracker poses of the first tracker (200) and the second tracker (300) in 5 DOF; and
determining (840), from the tracker poses and based on the registration of the tracking coordinate system with the image coordinate system, poses of the first vertebra (210) and the second vertebra (310) in 5 DOF.

2. The method (800) according to claim 1, wherein
each of the first and second tracker (200, 300) comprises an elongated body (220, 320) and wherein
the 5 DOF of the tracker poses exclude a DOF pertaining to a respective rotation of the first tracker (200) and the second tracker (300) with regard to a rotational axis defined by the elongated body (220, 320) of the respective tracker (200, 300).

3. The method (800) according to claim 1 or 2, wherein
at least one of the first and second tracker (200, 300) is an electromagnetic tracker (510, 520, 530) and the intraoperative tracking data comprise data from an device capable of processing an output signal of the at least one electromagnetic tracker (510, 520, 530).

4. The method (800) according to claim 2 or claim 3 when depending on claim 2, wherein
at least one of the first and second tracker (200, 300) comprises two imageable markers (230, 330) that are attached to the elongated body (220, 320) and spaced apart from each other along a length of the elongated body (220, 320), and
the intraoperative tracking data comprise second image data taken by a camera (710) of a tracking system (700) and indicative of the imaged markers (230, 330) of the first tracker (200) and the second tracker (300).

5. The method (800) according to claim 4, wherein
the respective two markers (230, 330) have the same mutual arrangement for each of the first tracker (200) and the second tracker (300), such that the first tracker (200) and the second tracker (300) cannot be differentiated in the image data solely by the imaged markers (230, 330).

6. The method (800) according to claim 4 or 5, wherein
a distance between the respective two markers (230, 330) along the length of the elongated body (220, 320) is different for the first tracker (200) and the second tracker (300).

7. The method (800) according to any of claims 4 to 6, wherein
at least one of the first tracker (200) and the second tracker (300) comprises a divot, and wherein the divot is configured to receive a tip of a screw; and, optionally comprising:
determining a length of the screw based on a distance between one of the imaged markers (230, 330) of the first or second tracker (200, 300) receiving the tip of the screw and at least one imaged marker attached to an instrument holding the screw.

8. The method (800) according to any one of claims 4 to 7, wherein
a virtual 6 DOF tracker comprising at least three of the imageable markers (230, 330) of the first and second tracker (200, 300) that are imaged in third image data taken by the camera (710) of the tracking system (700) is defined; and
wherein the tracking coordinate system is registered (810) with the image coordinate system using the at least three imageable markers (230, 330) of the virtual 6 DOF tracker as imaged in the third image data.

9. The method (800) according to any one of claims 1 to 7, wherein
a 6 DOF reference tracker (600) has a fixed relation to the patient and comprises at least three imageable markers (630) that are imaged in fourth image data taken by the camera (710) of the tracking system (700);
wherein the tracking coordinate system is registered (810) with the image coordinate system using the at least three imageable markers (630) of the 6 DOF reference tracker (600) as imaged in the fourth image data.

10. The method (800) according to claim 9, further comprising:
determining a change of the pose of at least one of the first and the second tracker (200, 300) relative to a pose of the reference tracker (600).

11. The method (800) according to any preceding claim, comprising determining a change of a pose of the first tracker (200) relative to a pose of the second tracker (300).

12. The method (800) according to any preceding claim, wherein
the first and second tracker (200, 300) each comprises an optically, in particular visually detectable identification characteristic (240, 340), wherein the identification characteristics (240, 340) of the first and second tracker (200, 300) are distinguishable from each other, and wherein the method (800) further comprises:
identifying at least one of the first tracker (200) and the second tracker (300) based on its identification characteristic (240, 340).

13. The method (800) according to claim 12 when depending at least on claim 2, wherein
the identification characteristics (240, 340) comprise an optically detectable surface characteristic (240, 340) of the first tracker (200) and second tracker (300), in particular of the respective elongated body (220, 320), and, optionally, wherein the optically detectable surface characteristics (240, 340) comprise different colors and/or different patterns.

14. The method (800) according to any preceding claim, comprising defining, based on the determined poses of the first vertebra (210) and the second vertebra (310) in 5 DOF, a trajectory for guiding a surgical tool (500).

15. The method (800) according to any preceding claim, further comprising:
visualizing the determined pose of the first and second vertebra (210, 310), or information derived therefrom.

16. The method (800) according to claim 15, wherein the visualizing comprises:
obtaining a first image data segment including the first vertebra (210) and a second image data segment including the second vertebra (310); and
arranging the first image data segment relative to the second image data segment based on the determined poses of the first vertebra (210) and the second vertebra (310) in 5 DOF.

17. A computer program product comprising instructions configured to perform the steps of the method (800) of any of claims 1 to 16 when the computer program product is executed on one or more processors.

18. A data processing system (720) comprising a processor (722) configured to perform the steps of the method (800) of any of claims 1 to 16.

19. A system (100) for determining the poses of at least two vertebrae of a patient, the system comprising:
a first tracker (200) trackable in 5 degrees of freedom, DOF, and attachable to a first vertebra (210) and a second tracker (300) trackable in 5 DOF and attachable to a second vertebra (310), wherein the first and second tracker (200, 300) each comprises an elongated body (220, 320) and two imageable markers (230, 330) that are attached to the elongated body (220, 320) and spaced apart from each other along a length of the elongated body (220, 320), wherein the respective two markers (230, 330) have the same mutual arrangement for each of the first tracker (200) and the second tracker (300), such that the first tracker (200) and the second tracker (300) cannot be differentiated in image data solely by the imaged markers (230, 330), wherein
the first and second tracker (200, 300) each comprises an optically detectable identification characteristic (240, 340), wherein the optically detectable identification characteristics (240, 340) of the first and second tracker (200, 300) are optically distinguishable from each other.

20. The system according to claim 19, further comprising:
a reference tracker (600) attachable to the patient, wherein the reference tracker (600) comprises at least three imageable markers (630).

## Amended claims

### Amended claims in accordance with Rule 137(2) EPC.

1. A computer-implemented method (800) of determining poses of at least two vertebrae of a patient when
a first tracker (200) trackable in 5 degrees of freedom, DOF, is attached to a first vertebra (210) and a second tracker (300) trackable in 5 DOF is attached to a second vertebra (310),
wherein each of the first and second tracker (200, 300) comprises an elongated body (220, 320),
wherein a tracking coordinate system is registered (810) in 6 DOF with an image coordinate system associated with first image data taken by a medical imaging system and indicative of the first and second vertebra (210, 310),
the method (800) comprising:
receiving (820) intraoperative tracking data;
determining (830), from the intraoperative tracking data, tracker poses of the first tracker (200) and the second tracker (300) in 5 DOF;
determining (840), from the tracker poses and based on the registration of the tracking coordinate system with the image coordinate system, poses of the first vertebra (210) and the second vertebra (310) in 5 DOF; wherein
**characterized in that**
the 5 DOF of the tracker poses exclude a DOF pertaining to a respective rotation of the first tracker (200) and the second tracker (300) with regard to a rotational axis defined by the elongated body (220, 320) of the respective tracker (200, 300).

2. The method (800) according to claim 1, wherein
at least one of the first and second tracker (200, 300) is an electromagnetic tracker (510, 520, 530) and the intraoperative tracking data comprise data from a device capable of processing an output signal of the at least one electromagnetic tracker (510, 520, 530).

3. The method (800) according to claim 1 or claim 2, wherein
at least one of the first and second tracker (200, 300) comprises two imageable markers (230, 330) that are attached to the elongated body (220, 320) and spaced apart from each other along a length of the elongated body (220, 320), and
the intraoperative tracking data comprise second image data taken by a camera (710) of a tracking system (700) and indicative of the imaged markers (230, 330) of the first tracker (200) and the second tracker (300).

4. The method (800) according to claim 3, wherein
the respective two markers (230, 330) have the same mutual arrangement for each of the first tracker (200) and the second tracker (300), such that the first tracker (200) and the second tracker (300) cannot be differentiated in the second image data solely by the imaged markers (230, 330).

5. The method (800) according to claim 3, wherein
a distance between the respective two markers (230, 330) along the length of the elongated body (220, 320) is different for the first tracker (200) and the second tracker (300).

6. The method (800) according to any of claims 3 to 5, wherein
at least one of the first tracker (200) and the second tracker (300) comprises a divot, and wherein the divot is configured to receive a tip of a screw; and, optionally comprising:
determining a length of the screw based on a distance between one of the imaged markers (230, 330) of the first or second tracker (200, 300) receiving the tip of the screw and at least one imaged marker attached to an instrument holding the screw.

7. The method (800) according to any one of claims 3 to 6, wherein
a virtual 6 DOF tracker comprising at least three of the imageable markers (230, 330) of the first and second tracker (200, 300) that are imaged in third image data taken by the camera (710) of the tracking system (700) is defined; and
wherein the tracking coordinate system is registered (810) with the image coordinate system using the at least three imageable markers (230, 330) of the virtual 6 DOF tracker as imaged in the third image data.

8. The method (800) according to any one of claims 1 to 6, wherein
a 6 DOF reference tracker (600) has a fixed relation to the patient and comprises at least three imageable markers (630) that are imaged in fourth image data taken by the camera (710) of the tracking system (700);
wherein the tracking coordinate system is registered (810) with the image coordinate system using the at least three imageable markers (630) of the 6 DOF reference tracker (600) as imaged in the fourth image data.

9. The method (800) according to claim 8, further comprising:
determining a change of the pose of at least one of the first and the second tracker (200, 300) relative to a pose of the reference tracker (600).

10. The method (800) according to any preceding claim, comprising determining a change of a pose of the first tracker (200) relative to a pose of the second tracker (300).

11. The method (800) according to any preceding claim, wherein
the first and second tracker (200, 300) each comprises an optically, in particular visually detectable identification characteristic (240, 340), wherein the identification characteristics (240, 340) of the first and second tracker (200, 300) are distinguishable from each other, and wherein the method (800) further comprises:
identifying at least one of the first tracker (200) and the second tracker (300) based on its identification characteristic (240, 340).

12. The method (800) according to claim 11 when depending at least on claim 2, wherein
the identification characteristics (240, 340) comprise an optically detectable surface characteristic (240, 340) of the first tracker (200) and second tracker (300), in particular of the respective elongated body (220, 320), and, optionally, wherein the optically detectable surface characteristics (240, 340) comprise different colors and/or different patterns.

13. The method (800) according to any preceding claim, comprising defining, based on the determined poses of the first vertebra (210) and the second vertebra (310) in 5 DOF, a trajectory for guiding a surgical tool (500).

14. The method (800) according to any preceding claim, further comprising:
visualizing the determined pose of the first and second vertebra (210, 310), or information derived therefrom.

15. The method (800) according to claim 14, wherein the visualizing comprises:
obtaining a first image data segment including the first vertebra (210) and a second image data segment including the second vertebra (310); and
arranging the first image data segment relative to the second image data segment based on the determined poses of the first vertebra (210) and the second vertebra (310) in 5 DOF.

16. A computer program product comprising instructions configured to perform the steps of the method (800) of any of claims 1 to 15 when the computer program product is executed on one or more processors.

17. A data processing system (720) comprising a processor (722) configured to perform the steps of the method (800) of any of claims 1 to 15.

18. A system (100) for determining the poses of at least two vertebrae of a patient, the system comprising:
a first tracker (200) trackable in 5 degrees of freedom, DOF, and attachable to a first vertebra (210) and a second tracker (300) trackable in 5 DOF and attachable to a second vertebra (310), wherein the first and second tracker (200, 300) each comprises an elongated body (220, 320) and two imageable markers (230, 330) that are attached to the elongated body (220, 320) and spaced apart from each other along a length of the elongated body (220, 320), wherein the respective two markers (230, 330) have the same mutual arrangement for each of the first tracker (200) and the second tracker (300), such that the first tracker (200) and the second tracker (300) cannot be differentiated in image data solely by the imaged markers (230, 330), wherein
the first and second tracker (200, 300) each comprises an optically detectable identification characteristic (240, 340), wherein the optically detectable identification characteristics (240, 340) of the first and second tracker (200, 300) are optically distinguishable from each other.

19. The system according to claim 18, further comprising:
a reference tracker (600) attachable to the patient, wherein the reference tracker (600) comprises at least three imageable markers (630).
